# EUROPEAN PATENT APPLICATION

(11) **EP 3 945 735 A1**
(43) Date of publication of application: **02.02.2022**
(21) Application number: 20207468.8
(22) Date of filing: 13.11.2020
(51) Int. Cl.: H04S 7/00

(54) **SOUND MANAGEMENT IN AN OPERATING ROOM**

(30) Priority: 30.07.2020 US 202063058897 P
(71) Applicant: Koninklijke Philips N.V., 5656 AG Eindhoven (NL)
(72) Inventor: FLEXMAN, Molly Lara, 5656 AE Eindhoven (NL); PANSE, Ashish Sattyavrat, 5656 AE Eindhoven (NL)
(74) Representative: Philips Intellectual Property & Standards

(57) **Abstract**

The present invention relates to sound management in an operating room. In order to improve the noise situation for staff during medical procedures, a device for sound management in an operating room is provided that comprises an input, a processor and a sound generation output. Besides audio data from at least one microphone positioned in the operating room receiving audio input of the operating room, also environmental context information of sound sources within the operating room is provided together with priority weighting for the sound sources within the operating room for at least one user. Sound parts of the audio data are identified and assigned to the sound sources based on the environmental context information. At least some of the sound parts are modified based on the priority weighting for the at least one user. The modified sound parts are provided for sound generation to the at least one user.

## Description

### FIELD OF THE INVENTION

The present invention relates to a device for sound management in an operating room, to a system for sound management in an operating room and to a method for sound management in an operating room.

### BACKGROUND OF THE INVENTION

During medical procedures, for example during examinations, interventions or operations, multiple tasks are performed by different individuals, comprising e.g. nurses, surgeons and technicians, and various devices are used. This complex acoustic situation results in a rather high level of noise in the operating room. That noise is comprised of background sound (e.g. forced air or fans on equipment), alerts (e.g. patient monitor heart rate), conversations (e.g. between staff members, sharing information for reporting), tools (e.g. surgical tools hitting the metal pans), and surgical sounds (e.g. aspiration). The key technical problem is that while operation room staff can attempt to reduce noise, the majority of it cannot be silenced completely (e.g. background noise, conversations, etc.). Hence, the different individuals in the operating room are exposed to the noise and have to mentally filter important from unimportant information. Noise-cancelling headphones may be used providing a reduced sound exposure. US 2012/0215519 A1 provides active noise cancellation comprising a spatially selective processing operation on a multichannel signal to separate a source component from a background component. WO 2016/131064 A1 provides a hearing aid comprising augmenting the audio area of interest by determining head position and/or gaze of the user. However, it has been shown that due to an increase of the complexity of staff and equipment during medical procedures, the overall noise further increases.

### SUMMARY OF THE INVENTION

There may thus be a further need to improve the noise situation for staff during medical procedures in an operation room.

The object of the present invention is solved by the subject-matter of the independent claims; further embodiments are incorporated in the dependent claims. It should be noted that the following described aspects of the invention apply also for the device for sound management in an operating room, for the system for sound management in an operating room and for the method for sound management in an operating room.

According to the present invention, a device for sound management in an operating room is provided. The device comprises an input, a processor and a sound generation output. The input is configured to provide audio data from at least one microphone positioned in the operating room receiving audio input of the operating room. The input is also configured to provide environmental context information of sound sources within the operating room. The input is further configured to provide priority weighting for the sound sources within the operating room for at least one user. The processor is configured to identify and assign sound parts of the audio data to the sound sources based on the environmental context information. The processor is also configured to modify at least some of the sound parts based on the priority weighting for the at least one user. The sound generator output is configured to provide the modified sound parts for sound generation to the at least one user.

This provides an augmented reality experience to the user in form of the acoustic scenario in the room overlaid with the modified sound parts, thus improving the noise situation to the user.

As a result, an intelligent way for sound management is provided that can benefits from additional contextual information.

According to an example, the environmental context information of the sound sources within the operating room comprises at least one of the group of spatial information of the sound sources in the operating room, visual appearance of sound sources in the operating room, type of sound sources in the operating room, content of the sound from the sound sources in the operating room, identifications of persons in the operating room that may act as sound sources in the operating room, possible signals and to-be-expected (expectable) signals from sound sources in the operating room.

According to the present invention, also a system for sound management in an operating room is provided. The system comprises at least one microphone configured to receive audio input from the operating room and to provide audio data from the operating room. The system also comprises at least one device for sound management in an operating room according to one of the preceding examples. The system further comprises at least one sound generation device. The at least one microphone provides the audio data to the input of the at least one device for sound management. The sound generator output provides the modified sound parts to the sound generation device of the at least one device for sound management to provide the modified sound to the at least one user.

According to an example, the environmental context information comprises at least a partial mesh of the room. The mesh comprises identifiable objects within the operating room.

According to an example, at least one spatial sensor arrangement is provided configured to provide spatial data of a user's surrounding in the operating room. The spatial data is suitable to generate the at least partial mesh of the room.

According to an option, the at least one spatial sensor arrangement comprises at least one of the group of depth sensing cameras, optical cameras, location detectors, identification of other team members in the room, position determination devices of the user within the room and markers that identify specific locations or objects, wherein the markers are provided as at least one of the group of optical markers, geometric markers and spatial anchors.

According to an example, at least one head-mounted device is provided for the user. The head-mounted device comprises the sound generation device.

According to an example, an eye tracker is provided to determine the user's gaze. The user's gaze is provided as an input to determine preferred sound sources.

According to the present invention, also a method for sound management in an operating room is provided. The method comprises the following steps:
- providing audio data from at least one microphone positioned in the operating room receiving audio input of the operating room;
   providing environmental context information of sound sources within the operating room;
- providing priority weighting for the sound sources within the operating room for at least one user;
- identifying and assigning sound parts of the audio data to the sound sources based on the environmental context information;
- modifying at least some of the sound parts based on the priority weighting for the at least one user; and
- providing the modified sound parts for sound generation to the at least one user.

According to an aspect, the present invention provides to apply environmental context to determine intelligent sound mixing for the listener. In an example, sound is provided via a wearable augmented reality headset and the sound is modulated based on environmental context. An example for an application is augmented reality in interventional guided therapy. More examples can be found in augmented reality applications for other clinical purposes, like medical examinations and medical operations. Further examples can be found in non-medical applications such as equipment installation and servicing, and manufacturing.

According to an aspect, a device for sound management in an operating room is provided that comprises an input, a processor and a sound generation output. Besides audio data from at least one microphone positioned in the operating room receiving audio input of the operating room, also environmental context information of sound sources within the operating room is provided together with priority weighting for the sound sources within the operating room for at least one user. Sound parts of the audio data are identified and assigned to the sound sources based on the environmental context information. At least some of the sound parts are modified based on the priority weighting for the at least one user. The modified sound parts are provided for sound generation to the user.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic setup of an example of a device for sound management in an operating room.
Fig. 2 shows a schematic setup of an example of a system for sound management in an operating room.
Fig. 3 shows basic steps of an example of a method for sound management in an operating room.
Fig. 4 schematically shows an example of a head-mounted device configured for sound management in an operating room.
Fig. 5 shows an example of a partial mesh of the operating room.
Fig. 6 shows an example of a setup in an operating room.
Fig. 7 shows an example of a scenery in an operating room.
Fig. 8 shows another example of a scenery in an operating room.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

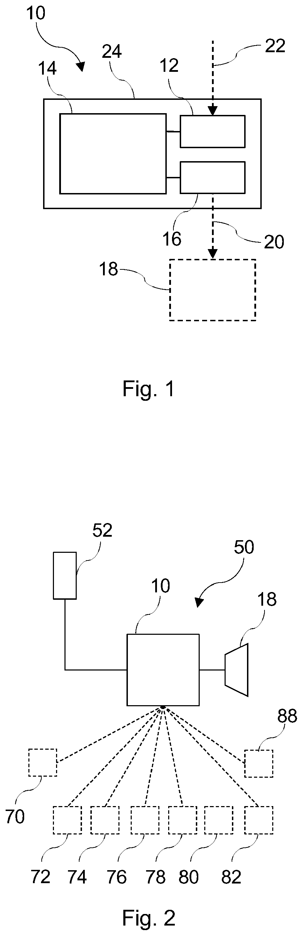

Fig. 1 shows a schematic setup of an example of a device 10 for sound management in an operating room. The device 10 comprises an input 12, a processor 14 and a sound generation output 16. The input 12 is configured to provide audio data from at least one microphone positioned in the operating room receiving audio input of the operating room. The input 12 is also configured to provide environmental context information of sound sources within the operating room. The input 12 is further configured to provide priority weighting for the sound sources within the operating room for at least one user. The processor 14 is configured to identify and assign sound parts of the audio data to the sound sources based on the environmental context information. The processor 14 is also configured to modify at least some of the sound parts based on the priority weighting for the at least one user. The sound generator output 16 is configured to provide the modified sound parts for sound generation to the at least one user.

As an option, a sound generator 18 is indicated. A data output connection 20 indicates the connection to the sound generator output 16.

A data input connection 22 indicates the connection from, for example, microphones to the input 12.

A frame 24 indicates that the input 12, the processor 14 and the sound generation output 16 can be provided in a common housing, for example as an integrated circuit arrangement. However, they might also be provided as separate components that are data connected to each other. In another option, portions of this controller may be in the cloud, such as the processor 14.

The device 10 for sound management can also be referred to as a controller for sound management in an operating room, or as sound controller. The device 10 provides augmented acoustic reality. The device 10 for sound management comprises rules and intelligent settings to provide adapted augmented sound to a user to improve the selected transfer of acoustic information to the user. The sound is modified based on knowledge of a location.

The input 12 refers to a data entry for providing the necessary data for the data processing. The input 12 can also be referred to as data entry or data interface to supply data to the processor 14. The input 12 can also be referred to as input unit or input interface.

The processor 14 refers to a data processing or computing component. The processor 14 provides the basis for the operation that uses data to determine modification for incoming sound data. The processor 14 can also be referred to as data processing unit or computation unit or central processing unit.

The sound generation output 16 refers to a data output for providing the modified sound parts. The sound generation output 16 can also be referred to as data exit or data interface to supply the modified sound data to a sound generation component. The sound generation output 16 can also be referred to as output unit or output interface.

The environmental context information relates to information about the surrounding of the user, i.e. the scenery within the operating room. This comprises spatial information of the possible sources, such as a mesh comprise of vertices, spatial anchors corresponding to sources, object recognition in one or more cameras, or the automated detection of sources from a combination of spatial anchors, mesh, and cameras.

The term "to identify sound parts" refers to determining data of sounds within the audio data that can be "segmented", i.e. distinguished form other sound parts and that can then be assigned to certain aspects of the environmental context information.

The term "to modify" relates to that at least some of the sound parts are amplified or suppressed or dampened. To modify also relates to an (audible) change of the sound characteristic of the sound parts, such as frequency modulation or the like.

The term "operating room" relates to a room or space in a hospital or other building for medical purposes, in which room or space operations take place. The term "operations" comprises all kinds of interventions and examinations, including interventional procedures as well as imaging procures and examining procedures. The term operating room thus also relates to imaging rooms, operating rooms, interventional suites, cathlabs, examination rooms, office-based labs (in hospitals and other buildings), ambulatory surgical centers and the like.

In an example, the priority weighting setting is provided as user priority weighting.
In a further example, the priority weighting setting is provided based on hospital rules.

In another example, the priority weighting setting is provided as algorithmically (e.g. via machine learning, deep learning, or other optimization techniques) generated priority weighting.

In an example, not further shown in details, the environmental context information of the sound sources within the operating room comprises at least one of the group of spatial information of the sound sources in the operating room, visual appearance of sound sources in the operating room, type of sound sources in the operating room, content of the sound from the sound sources in the operating room, identifications of persons in the operating room that may act as sound sources in the operating room and possible signals and to-be-expected (expectable) signals from sound sources in the operating room.

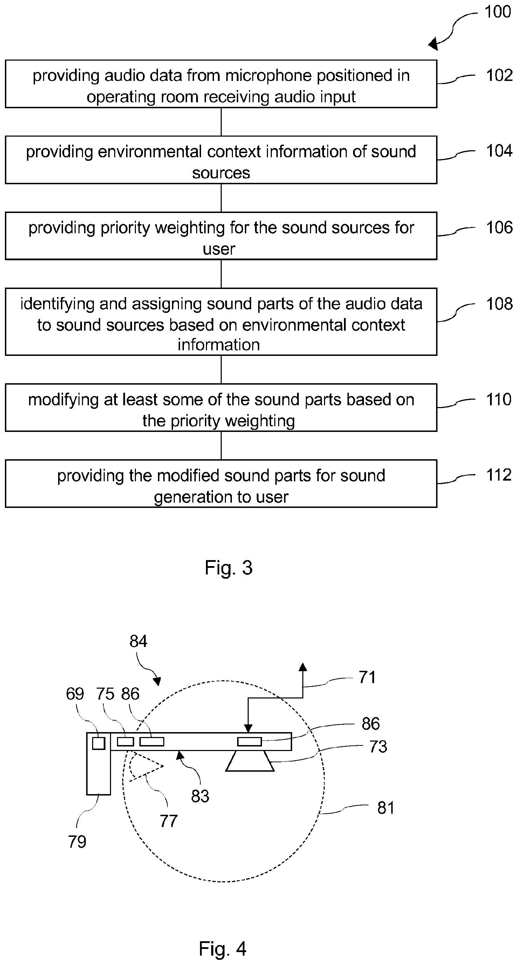

In an example, the environmental context information comprises a mesh of the room, wherein the mesh comprises vertices that delineate identifiable objects within the operating room (see also Fig. 4). The mesh of the room is also referred to as environmental mesh. The identifiable objects comprise at least one of the group of devices, appliances, equipment and subjects. In an example, the environmental context information comprises identified objects as preferred sound sources. The mesh provides a spatial map. The mesh may be provided as a sort of three-dimensional map reflecting essential objects and subjects within the operating room. In an example, spatial anchors are provided as a way to identify probable locations of objects. The mesh may be generated in real-time or periodically (e.g. at the beginning of the procedure) from one or more cameras, depth sensors, lidar, location sensors.

The term "subject" may also be referred to as individual. The "subject" may further also be referred to as patient, although it is noted that this term does not indicate whether any illness or disease is actually present with the subject.

Fig. 2 shows a schematic setup of an example of a system 50 for sound management in an operating room. The system 50 comprises at least one microphone 52 configured to receive audio input from the operating room and to provide audio data from the operating room. The system 50 also comprises at least one example of the device 10 for sound management in an operating room according to one of the preceding examples. The system 50 further comprises at least one sound generation device 54. The at least one microphone provides the audio data to the input 12 of the at least one device for sound management. The sound generator output 16 provides the modified sound parts to the sound generation device 54 of the at least one device for sound management to provide the modified sound to the at least one user.

In an example, the at least one microphone is positioned in the operating room.

In an example, the audio data comprises at least one of the group of object-based audio sounds (or object-generated, i.e. generated or caused by objects) and user-based audio sounds (or user-generated, i.e. generated or caused by users). The object-based audio sound is also referred to as object-generated sound, i.e. generated or caused by objects. The user-based audio sound is also referred to as user-generated sound, i.e. generated or caused by users.

In an example, the at least one microphone is positioned on the head mounted device.

Fig. 3 shows basic steps of an example of a method 100 for sound management in an operating room. The method 100 comprises the following steps: In a first step 102, also referred to as step a), audio data from at least one microphone positioned in the operating room receiving audio input of the operating room is provided. In a second step 104, also referred to as step b), environmental context information of sound sources within the operating room is provided. In a third step 106, also referred to as step c), priority weighting for the sound sources within the operating room for at least one user is provided. In a fourth step 108, also referred to as step d), sound parts of the audio data are identified and assigned to the sound sources based on the environmental context information. In a fifth step 110, also referred to as step e), at least some of the sound parts are modified based on the priority weighting for the at least one user. Further, in a sixth step 112, also referred to as step f), the modified sound parts are provided for sound generation to the at least one user.

The modified sound can also be referred to as targeted sound. The modifying of at least some of the sound parts comprises at least one of the group of amplification, suppression and change of the sound parts (e.g. band-pass filtering).

In an example, in addition to the modifying at least some of the sound parts, visual alerts (e.g. via the wearable augmented reality display) or tactile/haptic feedback (e.g. via a vibration in the wearable augmented reality display or in another wearable or hand-held device) or a combination of both is provided to the user. In an example, it is further provided the step of generating modified audio output for the at least one user overlaid to the audio input of the operating room providing augmented acoustic reality for the at least one user. In an option, the mesh is continuously updated and adapted according to changes in the spatial situation, such as moving staff members or moving of mobile equipment.

In an example, the environmental context information comprises at least a partial mesh of the room. The mesh comprises identifiable objects within the operating room.

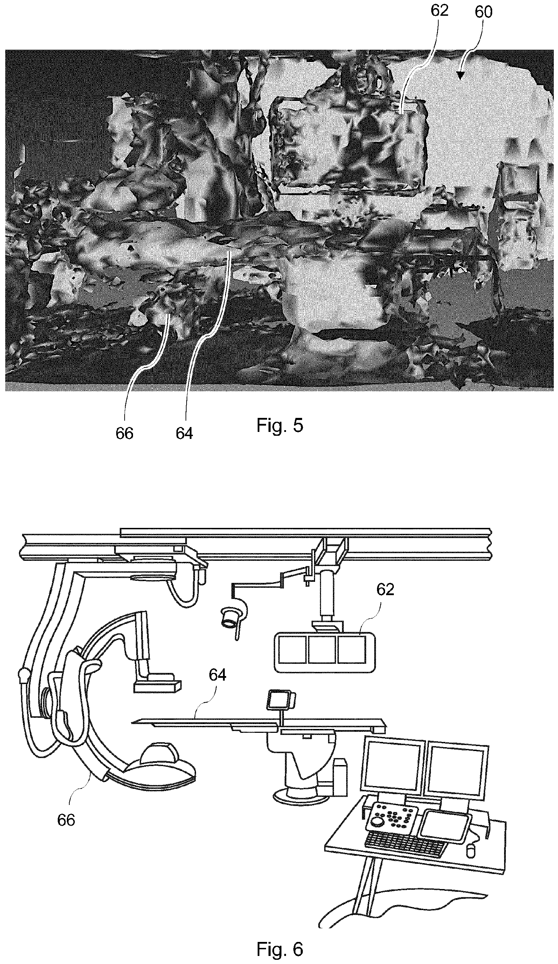

Fig. 5 shows an example of a partial mesh 60 of the operating room. For example, in the environmental mesh 60 at least one of the group of a monitor 62, a subject support 64 and an imaging system like a C-arm system 66 can be identified within the mesh 60.

Fig. 6 shows an example of a setup in an operating room for better understanding of Fig. 5. A similar setup is shown with an example of the monitor 62, an example of the subject support 64 and an example of the imaging system like the C-arm system 66. However, it is noted that the situation in Fig. 6 shows a slightly different arrangement and does not exactly match with the scenery in Fig. 5.

Referring back to Fig. 2, as an option, at least one spatial sensor arrangement 70 is provided configured to provide spatial data of a user's surrounding in the operating room. The spatial data is suitable to generate the at least partial mesh of the room. As an option, the at least one spatial sensor arrangement comprises at least one of the group of depth sensing cameras 72, optical cameras 74, markers 76 that identify specific locations or objects, wherein the markers are provided as at least one of the group of optical markers, geometric markers and spatial anchors, location detectors 78 and identification 80 of other team members in the room and position determination devices 82 of the user within the room.

In an option, encoders from actuated equipment is used to identify its location in the mesh, e.g. c-arm, robotic arm or robotic nurse.

In an example, at least one example of the depth sensing camera 72 is provided. In an example, the depth sensing cameras 72 are provided for near and far depth sensing. In an example, the optical cameras 74 are provided as forward & side facing cameras.

In an example, one or several meshes of the environment are generated by a separate mesh creator device (not shown in detail). For example, inputs may also come from one or more sets of sensors (e.g. one or more head wearable devices, one or more sensor bars mounted, or a combination).

In another example, one or several meshes of the environment are provided based on pre-operative data.

In a further example, one or several meshes of objects are provided.

In an example, object recognition is provided based on a camera feed.

In another example, object recognition is provided based on the mesh. For example, types of objects are identified based on the mesh structure and/or mesh segments.

In an example, the at least one spatial sensor arrangement comprises room-mounted devices, appliances or arrangements which comprise environment detection devices configured to provide data relating to the mesh of the room.

In another example, provided in addition or alternatively, the at least one spatial sensor arrangement comprises staff-mounted devices, appliances or arrangements which are comprising environment detection devices configured to provide data relating to the mesh of the room.

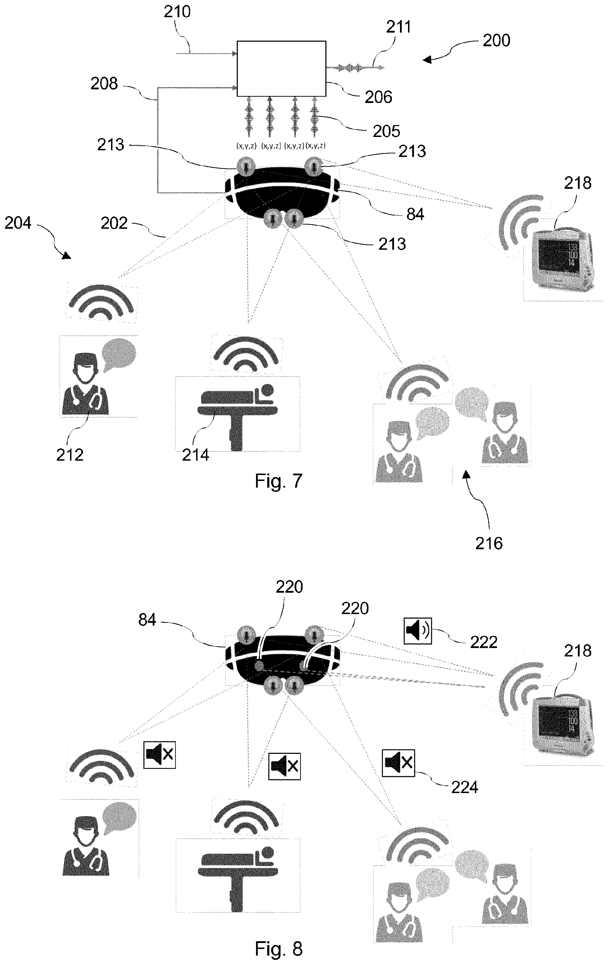

In an example, at least one head-mounted device 84 (see Fig. 7 and Fig. 8) is provided for the user. The head-mounted device 84 comprises the sound generation device 18.

Fig. 4 shows an example of the head-mounted device 84 configured for sound management in an operating room. The head-mounted device 84 comprises a frame structure 83 for wearing the head-mounted device 84 on a user's head 81. A lens structure 79 is provided to be arranged in viewing direction of the user, i.e. in front of the user's eyes 77. The lens structure 79 is provided such that the user can at least partly see through. A projection device 75 may be provided to provide projections on the lens structure 79 to provided visual augmented reality.

Further, the head-mounted device 84 comprises the sound generation device, for example loud speakers 73 in the area of the user's ears.

A data link 71 is indicated for connection with further components. For example, a central hub or console is provided for controlling several of the head-mounted devices 84.

In an example, the head-mounted device 84 is configured comprising the device 10 for sound management. For example, the processor 14 is integrated in the head-mounted device 84. In an example, the input 12, the processor 14 and the sound generation output 16 are provided separately, and the data link 71 provides the data connection, e.g. the data of the modified sound parts to the sound generation device like the loudspeaker 73.

In an example, further sensors 69, like a camera, are provided on the frame structure 83.

Further, also an eye tracker (not shown in detail in Fig. 4) may be provided to determine the user's gaze. Alternatively, other sensors (accelerometer, gyroscope, cameras, optical tracking) may provide input on user's head position which may be used as a surrogate for eye tracking.

In an example, one head-mounted device 84 is provided per user to provide user-specific modified sound. In an example, the head-mounted device 84 comprises a visual generation device (not shown in detail).

In an option, a plurality of the head-mounted devices 84 is provided and at least a part of the head-mounted devices 84 comprise environment detection devices configured to provide data relating to the mesh of the room.

In an example, the data is provided to create and/or update the mesh of the room.

The head-mounted devices 84 are used to commonly contribute to provide data which is used for generating and updating the mesh for the user.

Thus, a contribution from other users to the mesh is provided, for example, by adding data to the mesh from different positions (of the users) in the operating room.

In an example, the environmental context is determined using inputs from a wearable augmented reality headset that includes one or several of the group of depth sensing cameras (near and far), cameras (forward and side facing), meshes of the environment (generated by the augmented reality headset or known a priori), meshes of objects (generated by the augmented reality headset or known a priori), markers that identify specific locations or objects, location and identification of other team members in the room and determination of the position of the user within the room.

In an example, the environmental context information comprises identified objects as preferred sound sources. In an option, the preferred sound sources are identified by user input. In another option, provided in addition or alternatively to the option mentioned before, the preferred sound sources are predetermined for individual users in dependency of the current operating situation in the operating room.

In an example, one or more microphones 86 (see also Fig. 6) are provided that are configured to localize sound. The system is configured to register the localization of the sound to the mesh of the room.

For example, when the microphones and cameras generating the mesh are coming from the headset, this is maintained by a calibration of the mechanical relationship of the sensors within the device. If the microphones and cameras are not integrated together and are placed in the room, in an example, they are registered together.

In an example, an activity detector 88 is provided (see Fig. 2) to detect activity of a user. The user activity is used to modify the sound parts.

In an alternative example, the sound is modified or customized to a user regardless of the user activity.

In an example, an eye tracker 90 is provided (see Fig. 8) to determine the user's gaze. The user's gaze is provided as an input to determine preferred sound sources.

In an example, a sound controller for the operating room is provided that has one or more microphones receiving audio input and one or more speakers providing audio output. The audio output is modulated based on eye gaze.

The audio output can also be modulated based on duration of gaze. For example, duration of gaze on a particular audio input can then silence that input by "acknowledging" the alert.

In addition, audio modulating can be based on eye gaze towards a virtual object created from augmented reality that is a surrogate representative for a real object. For example, eye gaze towards a virtual screen of the patient monitor or a virtual warning message can acknowledge the alert similar to looking at the actual patient monitor.

Tracking the user's gaze provides the option to identify sound sources even when they are silent, i.e. when no sound is currently provided. The object (or subject) of interest can be identified to ensure that sound gets modified from this source once sound is created.

The provision of the gaze is based on the user looking at a scene and to use this information. As an example, gaze may provide depth information in addition to the mesh. Gaze provides an input of the intent of the user. Gaze can thus be used for identification of sound sources in addition or alternatively to the mesh. The gaze can therefore provide the environmental context information of sound sources within the operating room.

In an option, in addition to the modifying at least some of the sound parts, alert data is provided for a generation of visual alerts and/or for a generation of tactile/haptic feedback. In an example, a combination of both is provided.

In an example, the alert data is provided for the generation of the visual alerts by a visualizer (not shown in detail), for example, a pop up window to the user within the augmented reality wearable.

In another example, the alert data is provided for the generation of the tactile/haptic feedback by a vibrating element, for example a vibration element arranged in the headset or in a handle element (also not shown in detail).

Fig. 7 shows an example of a scenery in an operating room. A system 200 receives audio output 202 from multiple sources 204 in different locations in the room. Those locations as well as the sound 205 are input to a controller 206, along with additional information from augmented reality sensors 208 and a priori data 210. The controller then outputs modulated 3D audio output 211 that is fed back to the user. Multiple microphones 213 are provided. In the example of Fig. 7, the controller 206 receives input from four different sources in the room: a nurse 212 trying to talk to the operator, sounds from the surgical table 214, e.g. caused by an aspirator, two people 216 having a conversation in the corner of the room and an alarm 218 from the patient monitor. The controller 206 has an a-priori model (mesh & camera image) of the patient monitor and matches the alarm sound to that object. The rule specifies that these alerts are high priority and the sound is amplified. The two people having a conversation in the corner of the room are assigned to a low priority rule and thus the sound is suppressed. The sounds from the surgical table are mapped to that location in the room and are within a critical zone of the surgical area, therefore the sounds are left as is. In an option, the nurse is identified via facial recognition (from the camera) as a key staff member who is trying to talk to the operator. The speech is amplified for the user.

In a similar scenario, a different operator may have alternate rules, based on his or her role. For example, the nurse documenting the procedure may have a rule that removes the patient monitor alert as well as the sounds from the surgical site, in order to better hear the two different conversations that are taking place in the room. Similarly, the patient can also be wearing a headset and will only allow sound to pass through when speech is directed to the patient, as determined by the controller rules, an activation word, or environmental context.

The environmental context can also be extended to other types of context that may be relevant in the operating room, including: user context (from eye tracking, microphone, inward facing cameras of facial expression, gestures, head pose); system context (based on how the user is interacting with systems via the augmented reality wearable - for example, which screens are currently displayed, button presses on the virtual control panel); and/or clinical context (the type of procedure, the devices that are in use, the phase of the procedure, patient information).

Fig. 8 shows another example of a scenery in an operating room. As an option, eye gaze is used to selectively amplify and suppress different sources. For example, an eye gaze is detected by an eye gaze detector 220 to be focused on the alarm 218 from the patient monitor amplifies that sound and suppresses all other sound in the room.

A first symbol 222 indicates the targeted amplification as modification provided by the controller 206. A second symbol 224 indicates the targeted suppression as modification provided by the controller 206.

Since eye gaze can be a relatively noisy signal, it may also not perfectly map to the same location as the source of the sound. In that case, using information from the mesh of the room and the camera feed can map eye gaze to regions of the room that are also mapped to the source of sound.

Similarly, when attempting to amplify interpersonal communication, the eye gaze can be mapped to the location of the person that he or she is looking at. In this case the use of eye gaze can also modulate the target user's audio. Further, if two users are focused on the same object, their communication to each other can be amplified. The equipment is also linked to its operator. So, when the user is gazing at the equipment it selectively amplifies the sounds of both the equipment and any speech from the equipment operator.

This feature may be selectively enabled or disabled based on user preferences (e.g. pre-configured, enabled by a voice command, enabled after a certain dwell time, etc.).

In another example, a sound management system for an operating room is provided. The operating room comprises users and objects. The sound management system comprises one or more microphones positioned in the room for receiving audio input of the room, e.g. object audio sounds and users audio sounds. Further, one headset per user is provided having a microphone and speaker for receiving selective user audio and for outputting audio sounds specifically to the user. Still further, a prioritizer for making a priority list of audio sounds per user is provided. The sounds may be emphasized in the user headset. In an example, the list is made by a selection in the object sounds, user sounds and selective user audio sounds. Further, a mesh creator for creating a mesh of the room is provided. In an example, the mesh is provided as seen by the respective user. In another example, the mesh is provided by one sensor (e.g. camera) in a corner of the room. In another example, the mesh is provided by the user's headset, for example by each user headset. As an option, a means for detecting activity of each user is provided.

Further, a means is provided to selectively send audio sounds to each user, e.g. by a headset or speaker, based on information of his/her priority list, his/her mesh and optional his/her activity. Thus, the audio output provides a creating of sound by modulating the input sound based on environmental context.

In an example, a head-mounted display is provided that comprises at least one of the group of cameras, depth sensing cameras, microphones, speakers, head tracking, eye tracking and vibration elements. The cameras may be provided for visualization of the environment and simultaneous localization and mapping of the environment. The depth sensing cameras may be provided for environmental mapping and gesture recognition. The microphones may be provided for environmental sensing as well as voice recognition. The speakers may be provided for audio feedback. The head tracking may be provided for further adjusting the sound modification, such as enhancing audio signals from sources that are behind the user and thus not in the field of vision. The eye tracking may be provided for detecting gaze of the user. The vibration elements may be provided for haptic feedback, e.g. on the head mounted display, glasses or gloves worn by the user. Further, touch control of the head-mounted display may be provided. Still further, external devices may be provided, such as controllers, remote controls or keyboards.

In an example, the device for sound management, also referred to as controller, is implemented either directly on an augmented reality head mounted display or on a remote PC/Cloud that rapidly communicates back and forth with the head mounted display. In an example, the head mounted display has at minimum one microphone, but ideally more than one, in order to triangulate audio input to an approximate location in the room. That location and the sound profile are input to a controller. The controller also receives information from the augmented reality head mounted display such as the mesh of the room and one or more camera feeds. In addition, there may be a-priori information that is fed to the controller, such as the rules of the controller, 3D models and images of the different equipment, markers that are mapped to certain rules or equipment, photos of staff and their roles, etc.

The present invention provides a targeted augmented reality where a live acoustic field, also referred to as live sound stream, is supplemented with additional computer-generated acoustic information in form of the modified sound parts. The live acoustic stream (live sound stream) can be via the ear, headphones or other loudspeaker. This acoustic stream is augmented via the modified sound parts.

In an example, the augmented acoustic reality is provided by sound generation components next to the user's ears, or at least in an area close to the user's ears. For example, a head mounted display is provided with loudspeakers, e.g. earphones. Examples of head-mounted devices comprise wearables like the Google Glass or Microsoft's Hololens. Other examples of head-mounted devices are wearables by nReal, Meta, North, head-mounted display by Magic Leap, wearable headsets by Vusix and Bose augmented reality glasses.

Further, also additional augmentation can be provided in form of display, haptic and other feedback to the user. As an example, a head-mounted device is provided for each user that provides the augmented reality to the respective user. The present invention thus provides an implementation of augmented reality that adds feedback as virtual content to the real world.

In an example, the modified sound parts are provided to virtual reality, i.e. to a fully virtual acoustic world, or to mixed reality, i.e. a blending of real and virtual acoustic worlds.

According to an example, a computer program is provided enabling a processor to carry out the method of the preceding examples.

In an example, a computer program or program element for controlling an apparatus according to one of the examples above is provided, which program or program element, when being executed by a processing unit, is adapted to perform the method steps of one of the method examples above.

According to an example, a computer readable medium is provided having stored the program element of the preceding example.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for sound management in an operating room, the device comprising:
- an input (12);
- a processor (14); and
a sound generation output (16);
wherein the input is configured to:
- provide audio data from at least one microphone positioned in the operating room receiving audio input of the operating room;
- provide environmental context information of sound sources within the operating room; and
- provide priority weighting for the sound sources within the operating room for at least one user;
wherein the processor is configured to:
- identify and assign sound parts of the audio data to the sound sources based on the environmental context information; and
- modify at least some of the sound parts based on the priority weighting for the at least one user; and
wherein the sound generator output is configured to:
- provide the modified sound parts for sound generation to the at least one user.

2. Device according to claim 1, wherein the environmental context information of the sound sources within the operating room comprises at least one of the group of:
- spatial information of the sound sources in the operating room;
- visual appearance of sound sources in the operating room;
- type of sound sources in the operating room;
- content of the sound from the sound sources in the operating room;
- identifications of persons in the operating room that may act as sound sources in the operating room; and
- possible signals and to-be-expected (expectable) signals from sound sources in the operating room.

3. A system (50) for sound management in an operating room, the system comprising:
- at least one microphone (52) configured to receive audio input from the operating room and to provide audio data from the operating room;
- at least one device (10) for sound management in an operating room according to one of the preceding claims; and
- at least one sound generation device (18);
wherein the at least one microphone provides the audio data to the input of the at least one device for sound management; and
wherein the sound generator output provides the modified sound parts to the sound generation device of the at least one device for sound management to provide the modified sound to the at least one user.

4. System according to claim 3, wherein the environmental context information comprises at least a partial mesh of the room, wherein the mesh comprises identifiable objects within the operating room.

5. System according to claim 3 or 4, wherein at least one spatial sensor arrangement (70) is provided configured to provide spatial data of a user's surrounding in the operating room; wherein the spatial data is suitable to generate the at least partial mesh of the room; and
wherein the at least one spatial sensor arrangement comprises at least one of the group of:
- depth sensing cameras (72);
- optical cameras (74);
at least one of the group of optical markers, geometric markers and spatial anchors;
- location detectors (78) and identification (80) of other team members in the room; and
- position determination devices (82) of the user within the room.

6. System according to claim 3, 4 or 5, wherein at least one head-mounted device (84) is provided for the user; and
wherein the head-mounted device comprises the sound generation device.

7. System according to claim 6, wherein a plurality of head-mounted devices is provided; and
wherein at least a part of the head-mounted devices comprise environment detection devices configured to provide data relating to the mesh of the room.

8. System according to one of the claims 3 to 7, wherein the environmental context information comprises identified objects as preferred sound sources;
wherein, the preferred sound sources are identified by user input;
wherein, the preferred sound sources are predetermined for individual users in dependency of the current operating situation in the operating room.

9. System according to one of the claims 3 to 8, wherein one or more microphones are provided that are configured to localize sound; and
wherein system is configured to register the localization of the sound to the mesh of the room.

10. System according to one of the claims 3 to 9, wherein an activity detector (88) is provided to detect activity of a user; and
wherein the user activity is used to modify the sound parts.

11. System according to one of the claims 3 to 10, wherein an eye tracker (220) is provided to determine the user's gaze; and
wherein the user's gaze is provided as an input to determine preferred sound sources.

12. System according to one of the claims 3 to 11, wherein, in addition to the modifying at least some of the sound parts, alert data is provided for a generation of visual alerts and/or for a generation of tactile/haptic feedback.

13. A method (100) for sound management in an operating room, the method comprising the following steps:
- providing (102) audio data from at least one microphone positioned in the operating room receiving audio input of the operating room;
- providing (104) environmental context information of sound sources within the operating room;
- providing (106) priority weighting for the sound sources within the operating room for at least one user;
- identifying and assigning (108) sound parts of the audio data to the sound sources based on the environmental context information;
- modifying (10) at least some of the sound parts based on the priority weighting for the at least one user; and
- providing (112) the modified sound parts for sound generation to the at least one user.

14. A computer program enabling a processor to carry out the method of claim 13.

15. A computer readable medium having stored the program element of claim 14.
